# EUROPEAN PATENT SPECIFICATION

(11) **EP 2 583 248 B1**
(45) Date of publication and mention of the grant of the patent: **31.10.2018**
(21) Application number: 11795279.6
(22) Date of filing: 16.06.2011
(51) Int. Cl.: G06T 7/20, A61B 5/00

(54) **A METHOD AND A SYSTEM FOR MONITORING OF SLEEP AND OTHER PHYSIOLOGICAL CONDITIONS**
VERFAHREN UND SYSTEM ZUR ÜBERWACHUNG DES SCHLAFS UND ANDERER PHYSIOLOGISCHER ZUSTÄNDE
PROCÉDÉ ET SYSTÈME DE SURVEILLANCE DU SOMMEIL ET D'AUTRES CONDITIONS PHYSIOLOGIQUES

(30) Priority: 17.06.2010 US 355641 P
(43) Date of publication of application: 24.04.2013
(73) Proprietor: Card Guard Scientific Survival Ltd., 76305 Rehovot (IL)
(72) Inventor: GEVA, Nir, 74050 Nes Ziona (IL); GEVA, Yacov, London W1K 7DU (GB); TAL, Benny, 78374 Ashkelon (IL)
(74) Representative: Lehmbacher, Michael
(86) International application number: PCT/IB2011/052615
(87) International publication number: WO 2011/158198

(56) References cited:
- WO-A2-01/93756
- US-A1- 2004 010 390
- US-A1- 2005 027 207
- US-A1- 2007 156 060
- US-A1- 2007 249 952
- US-A1- 2008 228 045

## Description

### RELATED APPLICATIONS

This application claims priority from U.S. provisional patent application serial number 61/355,641 filing date June 17, 2010, entitled "A Systen and a Method for Monitoring of Sleep and Other Physiological Conditions".

### BACKGROUND OF THE INVENTION

In prior art solutions of monitoring, the monitored patient needs to be located and monitored in a facility in which gathering, analyzing and assessment of physiological monitoring information is possible. As some instances of monitoring are relatively long (e.g. a whole night, few days), it may be inconvenient and inefficient to hospitalize the patient for such long durations. Moreover, it means that monitoring can not be implemented in locations where highly trained personal (e.g. physicians) are unavailable.

Such locations may be, for example, a home of the patient, a nursing home, one of various hospitals departments that do not support monitoring of specific suspicious physiological conditions, etc.

There is therefore a need for a monitoring solution that counters these problems.

### BRIEF DESCRIPTION OF THE DRAWINGS

The invention is defined in the claims. However, both as to organization and method of operation, together with objects, features, and advantages thereof, may best be understood by reference to the following detailed description when read with the accompanying drawings in which:
Figures 1A and 1B illustrates various configurations of systems used for monitoring a physiological condition of one or more patients, according to various embodiments of the invention;
Figures 2A, 2B, and 3 illustrate monitoring systems, according to various embodiments of the invention;
Figure 4 illustrates a configuration of systems used for monitoring a physiological condition of a patient, according to an embodiment of the invention, and a monitoring system according to an embodiment of the invention;
Figure 5 illustrates positioning of a monitoring system and of its sensors, according to an embodiment of the invention;
Figure 6 illustrates a method for monitoring, according to an embodiment of the invention.
Figure 7 illustrates a monitoring control system, according to an embodiment of the invention.
Figure 8 illustrates a method for controlling monitoring, according to an embodiment of the invention; and
Figure 9 illustrates a method for monitoring, according to an embodiment of the invention.

It will be appreciated that for simplicity and clarity of illustration, elements shown in the figures have not necessarily been drawn to scale. For example, the dimensions of some of the elements may be exaggerated relative to other elements for clarity. Further, where considered appropriate, reference numerals may be repeated among the figures to indicate corresponding or analogous elements.

### SUMMARY OF THE INVENTION

According to an example a monitoring system is provided and may include: a transmitter configured to transmit to a monitoring control system physiological monitoring information that is responsive to information received from sensors that are carried by a patient; and a sensor manager configured to modify a monitoring state of a first sensor out of the sensors in response to instructions that are determined by the monitoring control system in response to an adequacy of the physiological monitoring information to monitoring of sleeping of the patient; wherein the transmitter is further configured to transmit to the monitoring control system physiological monitoring information responsive to information received from the first sensor after the modifying of its monitoring state.

The monitoring system may include a monitoring timing manager configured to cease, in response to a monitoring period decision that is made by the monitoring control system , a monitoring period that comprises collecting of the monitoring information transmitted to the monitoring control system ; wherein the monitoring period decision is made by the monitoring control system in response to an adequacy of the physiological monitoring information to monitoring of sleeping of the patient

The sensor manager may be configured to issue by an output interface of the monitoring system an instruction to relocate a sensor on the patient that corresponds to a received sensor relocating instruction that is issued by the monitoring control system in response to processing of the physiological monitoring information.

The monitoring system may include a that is video processor configured to process visual information of the patient that is received from a camera during a monitoring of the patient by at least one other sensor, wherein the transmitter is further configured to transmit to the monitoring control system the visual information of the patient.

The monitoring system may include an output interface configured to provide a questionnaire regarding a sleep affecting condition of the patient, and an input interface configured to receive at least one questionnaire result, wherein the transmitter is further configured to transmit to the monitoring control system questioner result information indicative of the at least one questionnaire result.

The transmitter is configured to transmit to the monitoring control system physiological monitoring information that is responsive to information received from the sensors that are of multiple types selected from a group of sensor types consisting of: electroencephalogram (EEG), electromyogram (EMG), electrocardiogram (ECG), electrooculography (EOG), and pulse sensor.

The transmitter is further configured to transmit to the monitoring control system location information that is responsive to information received from a positioning sensor and that is indicative of the relative location of at least one organ of the patient.

The system may include an interfering output interface, configured to awake the subject in response to a sleep related condition detected in response to the monitoring information.

A method for monitoring may be provided and may include carrying out by a monitoring system the method may include the following stages: transmitting to a monitoring control system physiological monitoring information that is responsive to information received from sensors that are carried by a patient; modifying a monitoring state of a first sensor out of the sensors in response to instructions that are determined by the monitoring control system in response to an adequacy of the physiological monitoring information to monitoring of sleeping of the patient; and transmitting to the monitoring control system physiological monitoring information responsive to information received from the first sensor after the modifying of its monitoring state.

The method may include ceasing a monitoring period that comprises collecting of the monitoring information transmitted to the monitoring control system in response to a monitoring period decision that is made by the monitoring control system in response to an adequacy of the physiological monitoring information to monitoring of sleeping of the patient

The method may include receiving a sensor relocating instruction that is issued by the monitoring control system in response to processing of the physiological monitoring information, and issuing a corresponding instruction to relocate a sensor on the patient by an output interface of the monitoring system.

The method may include transmitting to the monitoring control system visual information of the patient captured by a camera during a monitoring of the patient by at least one other sensor.

The method may include providing on an output interface of the monitoring system a questionnaire regarding a sleep affecting condition of the patient, receiving at least one questionnaire result by an input interface of the monitoring system, and transmitting to the monitoring control system questioner result information indicative of the at least one questionnaire result.

The transmitting of the physiological monitoring information may include transmitting to the monitoring control system physiological monitoring information that is responsive to information received from the sensors that are of multiple types selected from a group of sensor types consisting of: electroencephalogram (EEG), electromyogram (EMG), electrocardiogram (ECG), electrooculography (EOG), and pulse sensor.

The method may include transmitting to the monitoring control system location information that is responsive to information received from a positioning sensor and that is indicative of the relative location of at least one organ of the patient.

The method may include awaking the patient, by an interfering output interface, in response to a sleep related condition detected in response to the monitoring information.

A method for controlling monitoring may be provided and may include: receiving from a remote monitoring system physiological monitoring information that is responsive to information gathered by sensors that are carried by a patient; processing the physiological monitoring information for determining instructions for the monitoring system in response to an adequacy of the physiological monitoring information to monitoring of sleeping of the patient; issuing the instructions to the monitoring system; receiving from the monitoring system physiological monitoring information that is responsive to information received from a first sensor out of the sensors after the a monitoring state of the first sensor is modified by in response to the instructions; and analyzing sleep of the patient, wherein the analyzing comprises processing the physiological monitoring information that is received after the monitoring state of the first sensor is modified.

The method may include determining a monitoring period decision in response to an adequacy of the physiological monitoring information to monitoring of sleeping of the patient, wherein the monitoring period decision comprises timing information indicative of an end of monitoring period that comprises collecting of the monitoring information by the monitoring system.

The method may include issuing to the monitoring system, in response to processing of the physiological monitoring information, a sensor relocating instruction for relocating at least one of the sensors that gather information for the monitoring system.

The method may include providing monitoring information to a medical professional, receiving from the medical professional at least one instruction, and transmitting to the monitoring system an instruction to provide information on an output interface of the monitoring system in response to the instruction of the medical professional.

### DETAILED DESCRIPTION OF THE PRESENT INVENTION

In the following detailed description, numerous specific details are set forth in order to provide a thorough understanding of the invention. However, it will be understood by those skilled in the art that the present invention may be practiced without these specific details. In other instances, well-known methods, procedures, and components have not been described in detail so as not to obscure the present invention.

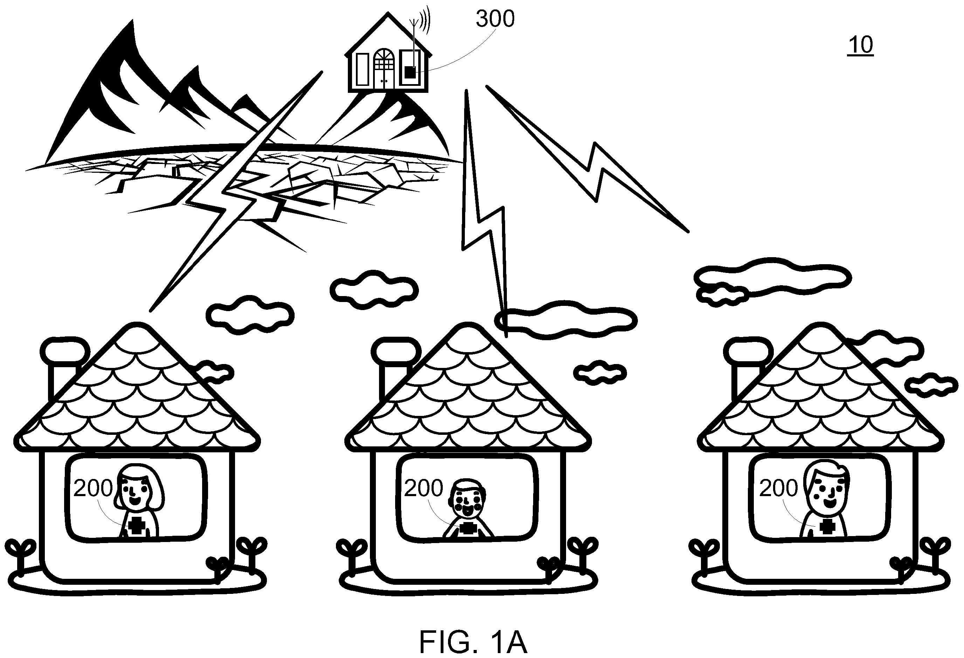

Figure 1A illustrates a configuration 10 of systems used for monitoring a physiological condition of one or more patients 100, according to an embodiment of the invention. It is noted that, according to an embodiment of the invention, that configuration may be used for monitoring of sleep of the patient, but this is not necessarily so - and other physiological conditions of the patient (e.g. heart condition, digestion, muscle tension, etc.) may also be monitored - possibly in addition to monitoring of sleep, but not necessarily so. A person who is skilled in the art would understand that the systems, methods, and configurations disclosed below may be implemented to physiological conditions other than sleep, even when the discussion exemplifies monitoring of sleeping conditions.

The configuration 10 includes one monitoring control system 300, that controls monitoring of physiological conditions of one or more patients 100, using one or more monitoring systems 200. It is noted that usually a different monitoring system 200 may be assigned to each patient 100, for monitoring her physiological condition, but this is not necessarily so, and according to an embodiment of the invention a single system 200 (or at least some components of which) may be used for the monitoring of physiological conditions of two or more patients.

Each monitoring system 200 usually collect information gathered by sensors that are either carried by the respective patient 100, or are located in the vicinity of that patient. Each monitoring system 200 than transmits to monitoring control system 300 physiological monitoring information that is responsive to information received from those sensors. This enables off-site assessment of the physiological monitoring information in a remote - and potentially central - location. In comparison to prior art solutions in which the patient needs to be located and monitored in a facility in which gathering, analyzing and assessment of physiological monitoring information is possible, the utilization of a remote monitoring control system 200 that receives the physiological monitoring information for processing enables monitoring of the patient 100 in a location in which analyzing and assessment of physiological monitoring information are not enabled.

For example, patient 100 may be located in her home, in a nursing home, in one of various hospitals departments that do not support monitoring of conditions that are implemented in systems 200 and 300 (e.g. a patient confined to an orthopedic department may suffer from sleep impediments which orthopedic department personnel have not the equipment or knowledge to analyze and assess).

Moreover, monitoring control system 300 may be supervised by one or more experts or experienced personnel (and/or by a dedicated computer program products), which can assess the process of monitoring being carried out by a remote monitoring system 200, and provide medical instruction (e.g. "patient 100 needs to be directed to a hospital immediately, or visited by a physician at home") and/or monitoring-process related instructions (e.g. "a camera should be turned on", "ECG electrode 4 should be relocated", "monitoring for additional 36 hours is required"). Such instructions - some of which may be followed by monitoring system 200 autonomously, some others may be followed by a non-specialist person - may be issued when monitoring system 200 does not have the means for issuing them (e.g. computational capabilities or software models), and/or an expert is not available in a location of patient 100.

Such configurations 10 may therefore provide financial saving, reduction in professional human resources required for monitoring of multiple patients, providing of advanced monitoring for patients 100 located away from dedicated monitoring centers (e.g. at home, and even in foreign countries), and so forth.

Various components of configuration 10 of systems used for monitoring a physiological condition of one or more patients 100 are exemplified in the following figures. It is however noted that the monitoring systems 200 utilized in configuration 10 may be different from the embodiments discussed below, and that the monitoring systems 200 discussed in relation to the following figures may be utilized in configurations other than configuration 10. Likewise, the monitoring control systems 300 utilized in configuration 10 may be different from the embodiments discussed below, and the monitoring control systems 300 discussed in relation to the following figures may be utilized in configurations other than configuration 10

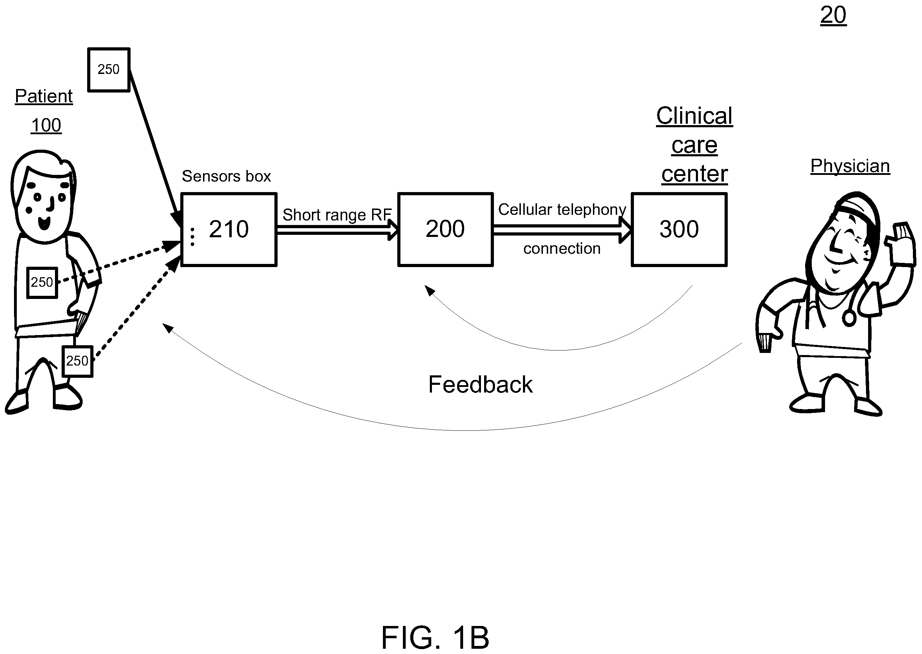

Figure 1B illustrates a configuration 20 of systems used for monitoring a physiological condition of one or more patients 100, according to an embodiment of the invention.

Multiple sensors 250 (that may and may not be a part of monitoring system 200) transmit data to system 200, usually via an intermediating component such as sensors manager 210 or equivalent component. It is noted that if sensors manager 210 is an external sensor manager 210 that is a stand alone unit in regards to other components of monitoring system 200 such as transmitter 230, it is also sometimes referred to as "sensor box". Sensors manager 210 may be a part of monitoring system 200 or external to it, and may be located on patient 100 (e.g. carried by it) or not. Also, some or all of the sensors 250 may be carried by patient 100. It is noted that some sensors 250 are adapted to detect information pertaining to the subject (also referred to as "the patient" in some contexts), while some other sensors 250 may possibly detect additional information (e.g. environmental data).

Information gathered by sensors 250 is transmitted to monitoring system 200 for real time monitoring and data recording - and potentially also for processing, wherein that transmission may be either wired or wireless (e.g. over radio frequency (RF) channel).

As aforementioned, monitoring system 200 transmits monitoring information (and especially physiological monitoring information) to monitoring control system 300, e.g. over a wireless channel such as cellular telephony connection.

According to an embodiment of the invention, monitoring control system 300 may be located in a clinical care center, or an equivalent institution, where a physician or other medically trained professional may review the data (possibly with assistance of computerized tools), and generate a medical report based on the physiological monitoring data. In monitoring control system 300, the information received from monitoring system 200 may be recorded, processed, analyzed, and used for decision making (e.g. by a physician).

It is noted that according to various embodiments of the invention, monitoring control system 300 may be a dedicated unit or a standard computing unit (e.g. a PC) with a dedicated software. According to an embodiment of the invention, monitoring control system 300 is an ACT4 monitor. According to an embodiment of the invention, monitoring control system 300 is located in a monitoring supervision center (e.g. a hospital). Such a monitoring supervision center may include one or more monitoring control system 300 for managing monitoring of multiple monitoring systems 200 used on multiple patients.

Conveniently, if there is any problem during the monitoring period, the monitoring supervision center (or at least monitoring control system 300) is aware of it within a short period (e.g. less than a second, few seconds, few minutes) because of the transmission of the monitoring information (and/or additional information such as system status report) and/or of processed information. Such a problem may be identified by monitoring system 200 and/or by monitoring control system 300.

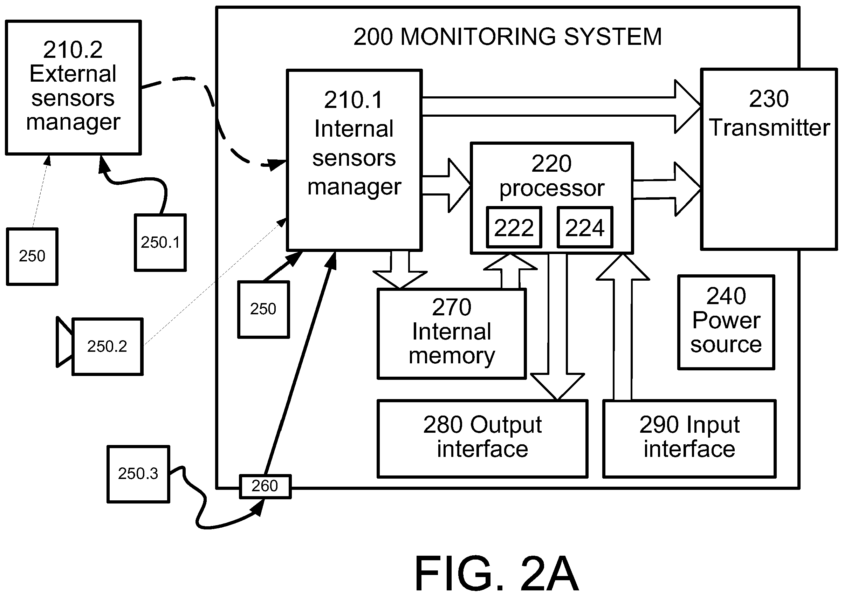

Figure 2A illustrates monitoring system 200, according to an embodiment of the invention. Monitoring system 200 includes sensor manager 210 and transmitter 230, and may also include other components such as integral monitoring information processor 220, power source 240, and so forth. It is noted that some of the sensors 250 may be integrated into monitoring system 200 (e.g. included within its casing), but this is not necessarily so.

Multiple sensors 250 are either included in monitoring system 200 (either in its casing or otherwise connected - e.g. by a fixed cable connection) or connected to which. It is noted that some sensors 250 (e.g. external sensors connected to monitoring system 200) may be detachably attached to monitoring system 200 in a physical connection (as well as, usually, an electronic and/or data connection). Such connections may be implemented, by way of example, using one or more sensor interfaces 260 (which may be standard interfaces - e.g. mini-USB, dedicated interfaces, and so forth). Some sensors 250 may be connected to monitoring system 200 over a wireless connection, transmitting and/or receiving information from monitoring system 200 over a wireless communication channel (e.g. Blue-tooth communication channel, RF channel, etc.). It is noted that some sensors 250 may be connected to a base that implements carrying by patient 100 (e.g. an adhesive surface, an elastic band, a connection pin, and so forth), but this is not necessarily so. Some sensors 250 may be carried by the patient 100 in an ad-hoc way, or not carried by her at all.

One or more sensor managers 210 of monitoring system 200 may conveniently be configured to manage the reception of information from the various sensors 250. It is noted that when multiple sensor managers 210 are implemented (internal sensor manager, denoted 210.1, and one or more external sensor managers, denoted 210.2), those sensor managers 210 may be organized in a hierarchical manner, but this is not necessarily so.

A sensor manager 210 may also be configured to otherwise manager sensors 250 that are connected to it - or which should be connected to it. For example, sensor manager may instruct (directly or indirectly) a sensor 250 to modify its state of monitoring (e.g. switch ON/OFF, modify its monitoring frequency, modify its monitoring parameters, etc.), and may inform another component of communication system 200 (and/or communication monitoring system 300) that a sensor 250 that is connected (or which should be connected) to it does not function as expected.

Information gathered by sensors 250 may be processed (at least a partial processing) by processor 220. It is noted that the processing by processor 220 of information gathered by sensors 250 is usually not parallel to a processing by monitoring control system 300 of physiological monitoring information that is transmitted to it by monitoring system 200. The processing by processor 220 of the information gathered by sensors 250 may be used, for example, for a preparation of physiological monitoring information from a raw pre-processed information gathered by sensors 250, before that information is transmitted to monitoring control system 300 for further processing and analysis.

It should be noted that, according to an embodiment of the invention, processor 220 may process also information other than physiological sensor information - e.g. information received from sensors 250 that does not pertain directly to the physiological condition of patient 100 (e.g. information pertaining to environmental condition), general information such as timing information, GPS derived information, information received from external sources such as monitoring control system 300, etc.

It is noted that, according to an embodiment of the invention, monitoring system 200 may include internal memory 270 that may be used for storing of information gathered by sensors 250 and/or for storing of information after its processing by processor 220. It should be noted that the monitoring may usually be for relatively long periods (e.g. hours, days, weeks), and that in some embodiments of the invention it may be inefficient and/or not desirable to store all the information gathered from sensors 250, from external systems, and/or processed information - internally. It is therefore noted that in some embodiments of the invention, a capacity of internal memory 270 is substantially smaller than the cumulative size of the information gathered by sensors 250 during a monitoring period in which patient 100 is monitored, and/or of the cumulative size of information that is transmitted to monitoring control system 300 and which pertains to that monitoring period. By way of example, the aforementioned amount of information gathered by sensors 250 and/or transmitted to monitoring control system 300 may exceed the capacity of internal memory 220, according to various embodiments of the invention, by one order of magnitude, by two orders of magnitudes, by three orders of magnitudes, by four orders of magnitudes, by five orders of magnitudes, by six orders of magnitudes, and so on. Therefore, a smaller memory is required in comparison to systems that collect all the information during the monitoring period.

It should be noted that monitoring system 200 may conveniently be a portable, patient-carried system, which is sufficiently light, durable, power-efficient, etc. to be carried by the patient (e.g. using a dedicated base such as an adhesive surface or a harness) for a prolonged durations of monitoring (e.g. hours, days, weeks). In other embodiments of the invention - only some components of monitoring system 200 are included in one or more such a patient-carried units, and other components of monitoring system 200 may be located away from patient 100 (e.g. next to its bed). It is noted that, according to an embodiment of the invention, monitoring system 200 is not carried by patient 100, and only some or all of the sensors 250 are carried by patient 100. It is noted that in various embodiments of invention (e.g. in configuration in which some components are carried by patient 100 and some are not), wired or wireless communication techniques may be implemented for communication between different components, as well as various combinations of wired and wireless communication.

Monitoring system 200 further includes transmitter 230 that is configured to transmit to monitoring control system 300 physiological monitoring information that is responsive to information received from sensors 250 that are carried by patient 100. It is noted that the physiological monitoring information transmitted to monitoring control system 300 may include some or all of the raw information received from sensors 250, and/or a processed version of it. It is further noted that, according to an embodiment of the invention, transmitter 230 may further provide to monitoring control system 300 physiological monitoring information responsive to information received from one or more sensors 250 that are not carried by patient 100 (e.g. a camera located on a tripod).

Transmitter 230 may work in various ways - according to various embodiments of the invention. For example, it may implement wireless (e.g. cellular) or wired (e.g. cable based internet) communication, may implement continuous, alternating, or burst sessions, and so forth.

It is noted that monitoring system 200 may transmit (e.g. wirelessly) to monitoring control system 300 different scopes of information in different embodiments of the invention and in different times. For example, monitoring system 200 may transmit immediately monitoring information (e.g. before analysis) and additional information at a later time. In another example, monitoring system 200 may transmit all (or vast majority) of the monitoring information at real-time or near real-time, and thus such data may be processed in monitoring control system 300 before it is fully processed by monitoring system 200, and even before monitoring system 200 gathers sufficient information for its internal processes.

It is further noted that the physiological monitoring information may be responsive to information received from combinations of various types of sensors 250 (and especially of physiological sensors 250) - such as the various types of sensors that are discussed below, in relation to different embodiments of the invention.

According to an example transmitter 230 may be further configured to transmit to monitoring control system 300 information other than physiological monitoring information - e.g. other monitoring information received from one or more sensors, timing information, operation status updates for monitoring system 200, etc. It is noted that in examples in which transmitter 230 transmits to monitoring control system 300 information in addition to the physiological monitoring information, such additional information may be combined with the physiological monitoring information, or submitted separately.

It is further noted that, according to an example transmitter 200 may transmit any of the above identified information to more than one monitoring control systems 300 (e.g. to one system that is located in a hospital where patient 100 is monitored, and to a national center), and/or to external systems other than monitoring control systems 300. In some examples transmission of information to one or more external system (e.g. monitoring control system 300) may be intermediated by another system (e.g. another monitoring system 200, another monitoring control system 300, a router, a gateway, etc.).

Monitoring system 200 may conveniently further include a receiver 232 (that may be integrated with transmitter 230 as a transceiver) from receiving information (e.g. instructions) from monitoring control system 300 or from other systems. It is noted that receiver 232 may be the one responsible for receiving information from one or more sensors - but it is not necessarily so.

As aforementioned, monitoring system 200 includes sensor manager 210, that in some examples may implement some or all of the functionalities discussed above. In some embodiments of the invention, sensor manager 210 is configured to modify a monitoring state of at least one first sensor (e.g. sensor 250.1) out of the sensors 250. Such modification may be implemented by a command to first sensor 250.1, may be implemented directly (e.g. by modifying a current applied to a sensor 250.1) and may be implemented by a person (either the patient 100 or someone else, e.g. a nurse) that receives such an instruction via an output interface of monitoring system 200 such as a screen or a speaker.

It is noted that sensor manager 210 may decide about the modification autonomously, and may modify (or instruct a modification) the state of first sensor 250.1 in response to instructions that are determined by processor 220 or by the monitoring control system 300 in response to an adequacy of the physiological monitoring information to monitoring of sleeping of the patient.

It is noted that the ability of sensors manager 210 to modify the monitoring state of one or more first sensors 250.1 in response to instructions received from monitoring control system 300, means that despite the fact that there is no physician or other expert medical professional that attends the monitoring of patient 100, in some embodiment such a person may still supervise the monitoring, determine that the information gathered by the sensors is not adequate, in give an instructions to modify a state of a sensor. Such a modification is also enabled by processing of the physiological monitoring data (as well as other information) by a superior processors, using superior software than the one available in monitoring system 200.

Examples for modifications in the monitoring state of first sensor 250.1 are operational mode (active, inactive, sleep, etc), monitoring frequency, monitoring parameters, etc.

It is noted that when such a modification in the state of one or more first sensors occur, transmitter 230 is further configured to transmit to monitoring control system 300 physiological monitoring information responsive to information received from first sensor 250.1 after the modifying of its monitoring state.

According to an example monitoring system 200 and monitoring control system 300 are configured to exchange data and commands which together are used to ensure that the system is configured properly, set-up properly, transmitting information properly, and so forth. Conveniently, monitoring control system 300 is configured to make certain that by the end of the monitoring period (and possibly during any shorter period within it), sufficient amount of usable monitoring data will be collected. This may require transmitting commands to change settings of monitoring system 200 and/or of the sensors 250, commands to retransmission of information, commands to inform the patient and/or the monitoring supervisor that some action need to be carried out (e.g. replacing non-functioning sensor, relocating a misallocated sensor, recharging of the system, etc.).

According to an example monitoring system 200 further includes a monitoring timing manager 222 (that may be a part of, e.g. processor 220) that is configured to modify the duration in which the monitoring of patient 100 is carried out (also referred to as monitoring period). According to an example monitoring timing manager 222 is configured to cease, in response to a monitoring period decision that is made by monitoring control system 300 (and/or by processor 220), a monitoring period that includes collecting of the monitoring information transmitted to monitoring control system 300; wherein the monitoring period decision is made by monitoring control system 300 (and/or by processor 220) in response to an adequacy of the physiological monitoring information to monitoring of sleeping of patient 100 (or of another physiological condition, e.g. as elaborated above).

According to an example sensor manager 210 is further configured to issue - by an output interface 280 of monitoring system 200 - an instruction to relocate a sensor 250 on the patient 100, wherein that instruction may correspond to a sensor relocating instruction received, that is issued by monitoring control system 300 (and/or by processor 220) in response to processing of the physiological monitoring information (and/or of additional information e.g. as exemplified above). For example, the instruction may be an instruction to relocate an ECG electrode that does not provide acceptable signal anymore.

It is noted that sensor manager 210 may be further configured, to issue - by an output interface 280 - instructions of other types that are intended to people (either the patient 100 or someone else such as a supervisor) that may relate to the operation of one or more sensors 250 (e.g. turn on or reposition a camera) or to other issues (e.g. instructions to call a doctor, to recharge monitoring system 200, to breath deeply, etc.). Such instructions as well may correspond to instructions received from monitoring control system 300 and/or from processor 220 in response to processing of the physiological monitoring information (and potentially of additional information as well).

According to an example monitoring system 200 further includes video processor 224 (that may be implemented by processor 220 or by another processor) configured to process visual information of the patient that is received from a camera (denoted 250.2) during a monitoring of patient 100 by at least one other sensor 250, wherein the transmitter 210 is further configured to transmit to monitoring control system 300 the visual information of the patient 100 (processed by video processor 224 or not). It is noted that indifferent embodiments of the invention, camera 250.2 may capture either video or still images, and transmitter 210 may transmit either video or still images.

Such a camera 250.2 may have different functions, such as capturing sensor positioning information of one or more sensors 250 (that may be used, by way of example, in deciding to instruct the patient 100 or the monitoring supervisor to relocating a sensor 250), for capturing video and/or still images of the patient 100 during one or more period (e.g. during sleep; such period may be determined using information received from other sensors).

Monitoring system 200 may further includes output interface 290 - e.g. a screen or a microphone.

According to an example output interface 290 may be configured to provide a questionnaire regarding a sleep affecting condition of patient 100. Such a questionnaire may be received in different examples from processor 220, from monitoring control system 300, and/or from memory 270, and may be responsive to monitoring information or not. According to an example the questionnaire may be at least partly determined by a physician operating monitoring control system 300, and may even include a communication between patient 100 and the physician or other trained personnel - e.g. via chat, voice communication etc.

According to such an example input interface 290 may be configured to receive at least one questionnaire result, wherein transmitter 230 is further configured to transmit to monitoring control system 300 questioner result information indicative of the at least one questionnaire result. Example to subjects that may be referred to in the questionnaire are perceptions of the patient 100, medications taken (e.g. before sleep, during the day), etc.

Monitoring system 200 may include various types of user input interface 290, such as operation control buttons, microphone, touch-screen, etc. According to an example at least one processor of the system is configured to operate and/or to modify its operation in response to signal received from the at least one user input interface.

According to an example transmitter 230 is configured to transmit to monitoring control system 300 physiological monitoring information that is responsive to information received from the sensors 250 that are of multiple types selected from a group of sensor types consisting of: electroencephalogram (EEG), electromyogram (EMG), electrocardiogram (ECG), electrooculography (EOG), and pulse sensor, microphone, etc. It is noted that other types of sensors 250 may also be implemented.

According to an example monitoring system 200 may further include a sensor 250 for detecting heartbeat information - e.g. heartbeat rhythm (or is configured to receive information from such external sensor). According to such an example the system may be configured to monitor two types of physiological situation (e.g. heart rate rhythm problems and sleeping condition).

According to an example transmitter 230 is further configured to transmit to monitoring control system 300 location information that is responsive to information received from a positioning sensor 250.3 and that is indicative of the relative location of at least one organ of the patient. Such location information may be received from a GPS, from an acceleration based sensor (indicative of movement), and so forth.

The location information may be used, for example, in a processing of the physiological monitoring information (e.g. ECG information) to determine if detected behavior (e.g. ECG morphological modification) may result from an angle or position in which the subject sleep or other medical or physiological situation may cause the detected behavior.

According to an example monitoring system 200 may further include an interfering output interface (not denoted, may be output interface 280 or another output interface) that is configured to awake patient 100 in response to a sleep related condition that is detected in response to the monitoring information. For example, such an interfering output interface may sound an alarm, may issue an electrical shock, etc.

According to an example the monitoring system 200 may include interaction capabilities (e.g. using the output interface 280 or the interfering output interface) for interacting with the subject - for example, for modifying a physiological condition of the subject. For example, according to an example monitoring system 200 may include means (e.g. speaker, electric shock generator, etc.) for awaking the subject if the subject falls asleep, shows symptom of acute tiredness, if sensors are not connected properly, and so on). It is noted that monitoring system 200 and/or monitoring control system 300 may determine that only some of the components of monitoring system 200 are not functioning as expected, and that no such action should be carried out (e.g. if the monitoring information gathered is sufficient).

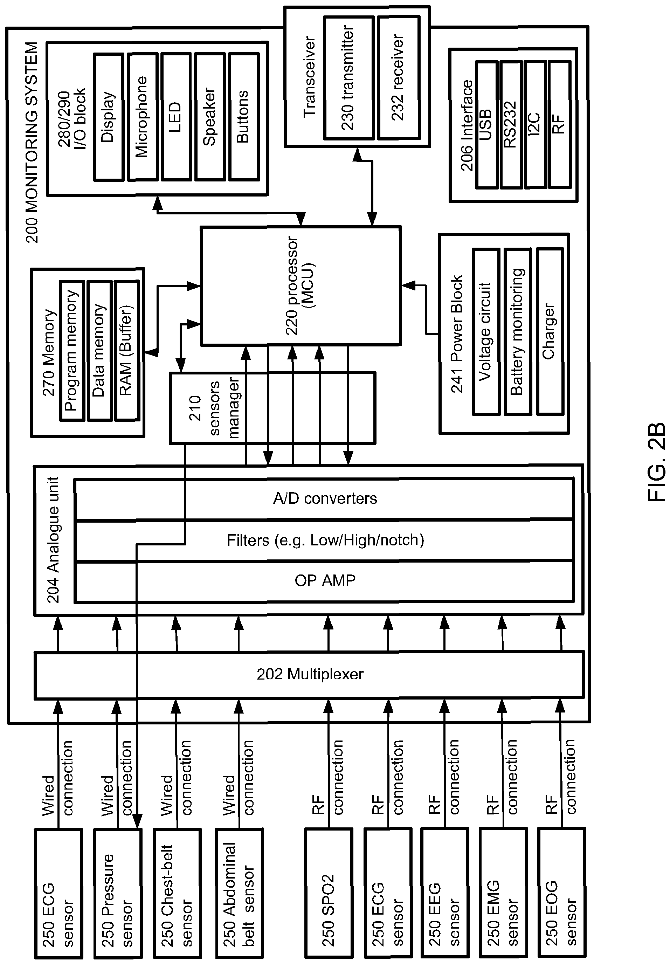

Figure 2B illustrates monitoring system 200, according to an example. Monitoring system 200 may include, according to various embodiments of the invention, various types of sensors such 250 as: ECG sensors, pressure sensors, chest belt sensor, abdominal belt sensors, SP02 sensor, EEG sensors, EMG sensors, EOG sensors, and so forth. Such sensors may transmit the monitored data to monitoring system 200 through some intermediating units, and either wirelessly (e.g. RF) or in a wired fashion.

The signals received by one or more of the sensors 250 may be multiplexed by a multiplexer 202 that multiplexes the various signals received from those sensors 250 (not necessarily all of the sensors) and transmits them to an analog unit 204 in which those signals are processed in an analog fashion (while it is noted that in other embodiments, digital signal processing may be implemented, and some or all of the sensors 250 may transmit information in a digital manner).

For example, in analog unit the signals may be amplified (e.g. by an operational amplifier, denoted "OP amplifier"), filtered by one or more filters, and converted to a digital signals, that is transmitted to other components such as processor 220.

It is noted that monitoring system 200 may include a power unit 241 that may include components such as internal power source 240 (not illustrated in figure 2B), voltage circuit, battery monitoring unit, charger, etc.). According to an example monitoring system 200 may receive power from an external source via a power interface - that may be used for reception of data as well in some embodiments - e.g. USB or mini-USB connection.

Monitoring system 200 may include various types of interfaces, such as USB, RS232, I2C, RF, collectively denoted 206. Monitoring system 200 may include different types of I/O components such as microphone, speaker, LED, buttons, etc. It is noted that the microphone, if implemented, may be used for recording of snoring and/or ambient noise, as well as for reception of voice commands, if implemented.

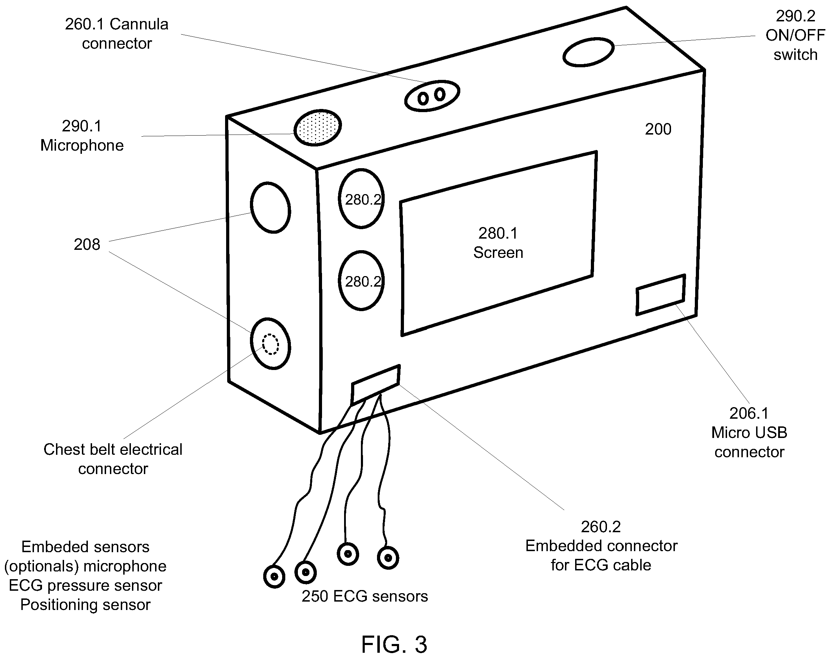

Figure 3 illustrates monitoring system 200, according to an example. Monitoring system 200 that is illustrated in figure 3 is intended to be carried by patient 100 during monitoring (e.g. at nights), and may include connectors for connecting to an elastic band, a harness, or an equivalent connecting means. For example, system 200 may include clasps connectors 208 for connecting to a chest belt.

Monitoring system 200 may include various output devices 280 such as screen 280.1 and speaker 280.2. Monitoring system 200 may also include one or more input devices such as microphone 290.1 and ON/OFF switch 290.2.

Monitoring system 200 may also include one or more sensor interfaces 260 for connection of various types of sensors, such as cannula connector 260.1, and embedded connector 260.2 for ECG cable. As aforementioned, monitoring system 200 may include various types of interface 206, such as micro USB connector 206.1, that may be used, inter alia, for charging of the internal power source 240 (battery) and for downloading and uploading of data, e.g. for software burning and updates.

As aforementioned, different embodiments of monitoring system 200 (and possibly also of monitoring control system 300) may include various systems for monitoring of various physiological conditions (e.g. sleep, tiredness, distraction, etc.). It should also be noted that different embodiments of the invention may also be implemented for different types of subjects (e.g. human, canine, bovine, etc.).

It is noted that according to an example at least some of sensors 250 may be remote from monitoring system 200 and have no direct physical contact with which (wherein communication cable may and not be used as the sole physical connection). Some of the sensors 250 that are carried by patient 100 may be located on a body of the patient while others may be or in close proximity to which (e.g. on a shirt of the subject, on an elastic band worn by the subject, on an adhesive surface attached to the patient, etc.).

Conveniently, the system enables monitoring in all the accepted levels (levels 1 through 4), but this is not necessarily so.

While monitoring system 200 may include internal power source 240, sensors 250 may be powered by sensor- included power source, from the power source 240 of monitoring system 200, from an external power source, etc. According to an example the system may also be connected to an external source, for continuous operation and/or for recharging.

According to an embodiment of the invention, monitoring system 200 (and/or monitoring control system 300) may combine analysis from various sources such as HRV received from ECG, or SP02, in order to perform additional analyses, e.g. in order to detect additional diseases (on top off - for example - sleeping problems), additional physiological and/or medical conditions, detecting possible causes for a detected physiological condition (e.g. sleeping problems), etc. Some conditions that may be detected (according to various embodiments of the invention) are diabetes, tension, heart condition, etc.

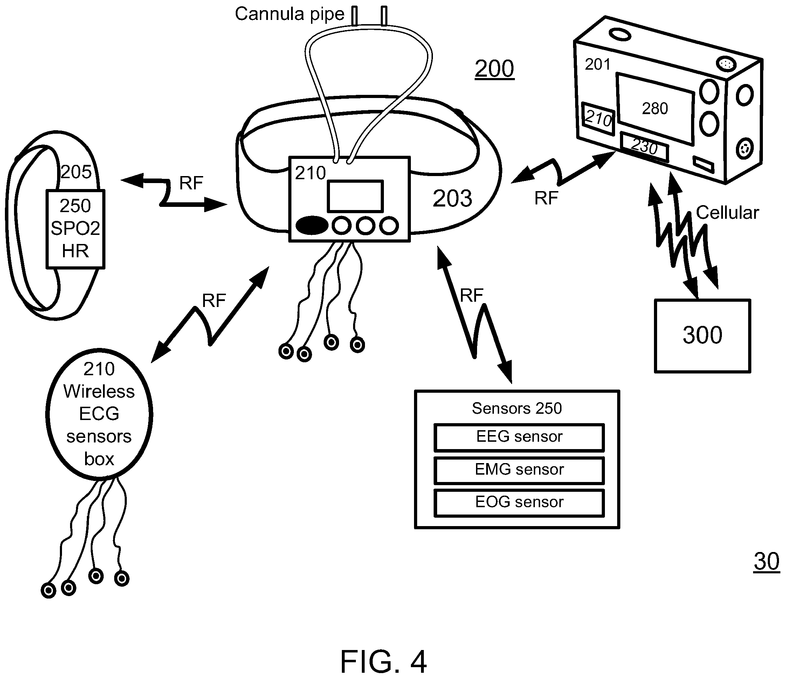

Figure 4 illustrates configuration 30 of systems used for monitoring a physiological condition of a patient 100, according to an example and an embodiment of monitoring system 200.

According to an example monitoring system 200 includes main unit 201 that may include components such as some or all of transmitter 230, power unit 240, processor 220, output interface 280, input interface 290, and so on. The main unit 201 receives information from sensors 250 via wired and/or wireless communication (e.g. RF) via other intermediating units - wherein communication from a sensor 250 may be intermediated more than once before arriving to main unit 201.

It is noted that, according to an example main unit 201 is not carried by the patient, and may be located, for example, in the same room as patient 100. According to an example main unit 201 (or other components of system 200) may be integrated into a computer (e.g. PC computer, lap-top computer, computer of a medical machine, PDA, and so on) and use facilities of that computer (e.g. processor, screen, keyboard, network connectivity) for implementing functionalities of monitoring system 200 such as those disclose in relation to the previous figures. For example, main unit 201 may be integrated into an RF capable USB dongle that may be inserted into a standard port of a personal computer. According to an example main unit 201 may be a software computer program installed into another component and utilizing its resources.

Monitoring system 200 may include components such as chest belt 203 that may include connections to some sensors 250 such as a cannula pipe and possibly also integrated ECG sensors. A sensors manager 210 located on chest belt 203 may receive information from sensors 250 connected to it, as well as from other sensors manager, e.g. a sensors manager (not illustrated) that is located on another elastic band 205 that includes SP02 sensor 250 as well as heart rate sensor 250, or from a sensors manager 210 of an optional wireless ECG sensors. It is noted that some sensors 250 (such as EEG, EMG and EOG sensors) may be united or share components.

According to an example monitoring system 200 (or at least some components of which) is integrated into or attached to a chest/abdomen belt or girdle (which may and may not be elastic) which can be worn by the patient 100, at least partially around the chest and at least partially around the abdomen. Other belts that may be used are abdomen belt and/or chest belt, among other types known in the art.

According to an example one or more ECG sensors 250 may be integrated into such a belt, which may turn, according to an embodiment of the invention, cumbersome cable connections that connects ECG sensors to the recording unit to unnecessary.

According to an example at least one processor 220 of monitoring system 200 (and/or of monitoring control system 300) is configured to process monitoring signals to validate quality of received signals. Such validation may be used for determining if there is a need to replace or relocate a sensor, to determine if there is a need to adjust the monitoring period or so on.

It is noted that according to an example the monitoring is carried out only in non-consecutive time periods (e.g. only during nights, from hour prior to going to sleep until waking up, etc.). In such situation, for example, processing of the monitoring information received from one or more sensors 250 may yield a decision that the patient 100 needs to be monitored for an additional night.

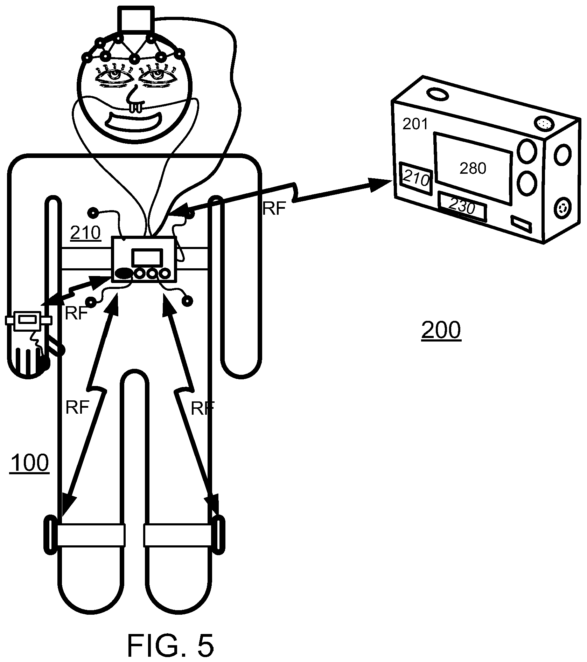

Figure 5 illustrates positioning of monitoring system 200 and of its sensors 250, according to an embodiment of the invention. As noted, various sensors 250 may be located on different parts of the patient 100- e.g. legs, hands, head, nose, chest, abdomen, etc. it is noted that, according to an example monitoring system 200 (or some components of it such as main unit 201) may be located in a distance from the patient 100 (e.g. in the same room), and not necessarily carried by her.

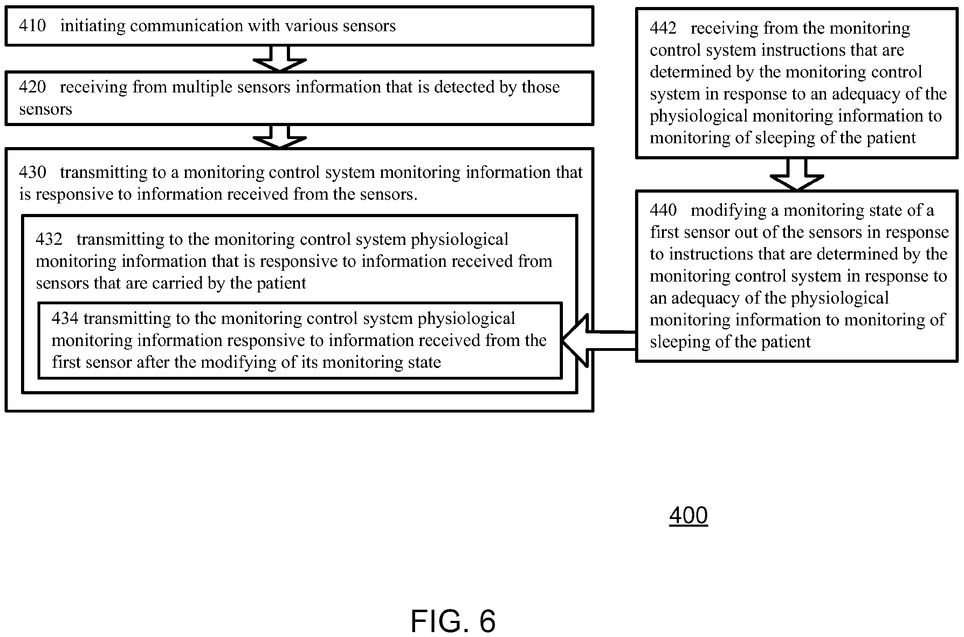

Figure 6 illustrates method 400 for monitoring, according to an example. Referring to the examples set forth in the previous drawings, it is noted that method 400 may be carried out by monitoring system 200, and that various embodiments of method 400 may implement all the functionalities of the various embodiments of monitoring system 200 discussed above, even if not explicitly elaborated. The various stages of method 400 are conveniently carried out by a monitoring system.

Method 400 may start with stage 410 of initiating communication with various sensors. Referring to the examples set forth in the previous drawings, the sensors may be sensors 250. It is noted that some of the sensors may be incorporated into the monitoring system, but not necessarily so. It is noted that additional instances of initiating communication with sensors may also be implemented when following stages of method 400 are carried out (e.g. if sensors are being added).

The method may also include stage 420 of receiving from multiple sensors information that is detected by those sensors. It is noted that conveniently, at least some of the sensors are physiological sensors, detecting information that pertain to a physiological condition of a patient.

Method 400 includes stage 430 of transmitting to a monitoring control system monitoring information that is responsive to information received from the sensors. Referring to the examples set forth in the previous drawings, stage 430 may include transmitting information to monitoring control system 300.

Stage 430 includes stage 432 of transmitting to the monitoring control system physiological monitoring information that is responsive to information received from sensors that are carried by the patient.

Method 400 further includes stage 440 of modifying a monitoring state of a first sensor out of the sensors in response to instructions that are determined by the monitoring control system in response to an adequacy of the physiological monitoring information to monitoring of sleeping of the patient. Stage 440 may be preceded by stage 442 of receiving the instructions from the monitoring control system.

Stage 440 is followed by stage 434 (that may be a part of stages 430 and possibly also 432) of transmitting to the monitoring control system physiological monitoring information responsive to information received from the first sensor after the modifying of its monitoring state.

According to an example method 400 further includes ceasing a monitoring period that includes collecting of the monitoring information transmitted to the monitoring control system in response to a monitoring period decision that is made by the monitoring control system in response to an adequacy of the physiological monitoring information to monitoring of sleeping of the patient.

According to an example method 400 further includes receiving a sensor relocating instruction that is issued by the monitoring control system in response to processing of the physiological monitoring information, and issuing a corresponding instruction to relocate a sensor on the patient by an output interface of the monitoring system.

According to an example method 400 further includes transmitting to the monitoring control system visual information of the patient captured by a camera during a monitoring of the patient by at least one other sensor. Such camera may have different functions, such as capturing sensor positioning information (helpful for instructing the subject/monitoring supervisor for relocating a sensor), for capturing video and/or still images of the subject during one or more period (e.g. during sleep; such period may be determined using information received from other sensors).

According to an example method 400 further includes providing on an output interface of the monitoring system a questionnaire regarding a sleep affecting condition of the patient, receiving at least one questionnaire result by an input interface of the monitoring system, and transmitting to the monitoring control system questioner result information indicative of the at least one questionnaire result.

According to an example the transmitting of the physiological monitoring information includes transmitting to the monitoring control system physiological monitoring information that is responsive to information received from the sensors that are of multiple types selected from a group of sensor types consisting of: electroencephalogram (EEG), electromyogram (EMG), electrocardiogram (ECG), electrooculography (EOG), and pulse sensor, microphone, etc.

According to an example method 400 further includes transmitting to the monitoring control system location information that is responsive to information received from a positioning sensor and that is indicative of the relative location of at least one organ of the patient.

According to an example further includes awaking the patient, by an interfering output interface, in response to a sleep related condition detected in response to the monitoring information.

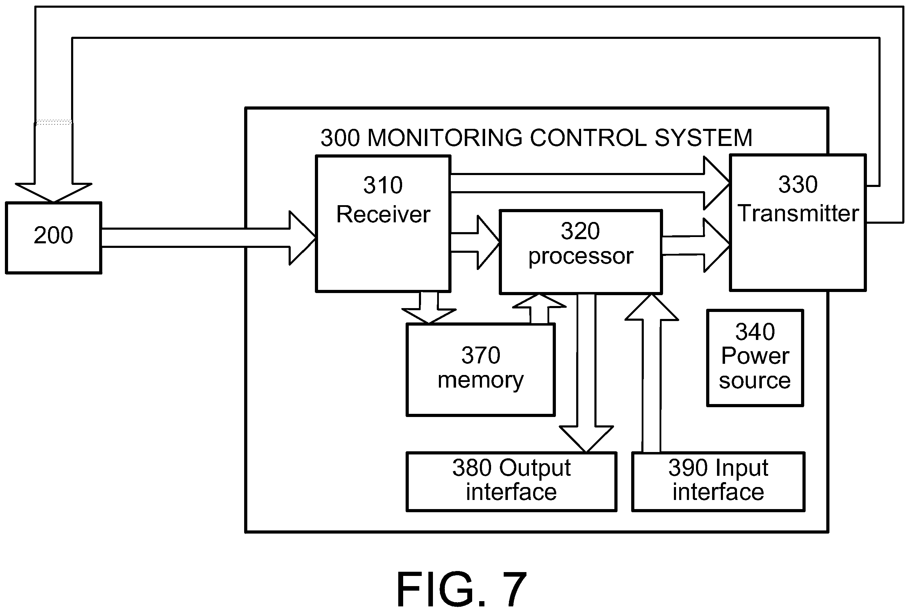

Figure 7 illustrates monitoring control system 300, according to an embodiment of the invention.

Monitoring control system 300 includes receiver 310 configured to receive from a remote monitoring system (such as monitoring system 200) physiological monitoring information that is responsive to information gathered by sensors (e.g. sensors 250) that are carried by a patient (e.g. patient 100). It is noted that monitoring control system 300 may receive information from more than one monitoring systems, and information that pertain to more than a single patient.

Monitoring control system 300 further includes a processor 320 that is configured to process the physiological monitoring information for determining instructions for the monitoring system in response to an adequacy of the physiological monitoring information to monitoring of sleeping of the patient (or of another physiological state of the patient).

Processor 320 is further configured to issue the instructions to the monitoring system, e.g. via a transmitter 330 of monitoring control system 300.

It is noted that receiver 310 is further configured to receive from the monitoring system physiological monitoring information that is responsive to information received from a first sensor out of the sensors after the a monitoring state of the first sensor is modified by in response to the instructions.

Processor 320 (or another processor of monitoring control system 300) is configured to analyze sleep of the patient (and/or another physiological state of which), wherein the analyzing includes processing the physiological monitoring information that is received after the monitoring state of the first sensor is modified. It is noted that the analyzing may include analyzing of physiological monitoring information received before or after modifications of one or more sensors, and may also include analyzing of non-physiological monitoring information and of other types of information as well.

Monitoring control system 300 may further include additional components such as a memory 370, an output interface 380, and input interface 390 (the last to may be used, by way of example, for communication with a physician that reviews and affects the analysis), a power source 340, and so forth.

It is noted that, according to an example monitoring control system 300 (or at least some components of which) may be integrated into a computer (e.g. PC computer, lap-top computer, computer of a medical machine, PDA, and so on) and use facilities of that computer (e.g. processor, screen, keyboard, network connectivity) for implementing functionalities of monitoring control system 300. For example, monitoring control system 300 may be integrated into a USB dongle that may be inserted into a standard port of a personal computer. According to an example monitoring control system 300 may be or may include a software computer program installed into another component and utilizing its resources.

According to an example processor 320 (or another processor of monitoring control system 300) is configured to determine a monitoring period decision in response to an adequacy of the physiological monitoring information to monitoring of sleeping of the patient, wherein the monitoring period decision includes timing information indicative of an end of monitoring period that comprises collecting of the monitoring information by the monitoring system.

According to an example monitoring control system 300 is further configured to issue to the monitoring system, in response to processing of the physiological monitoring information, a sensor relocating instruction for relocating at least one of the sensors that gather information for the monitoring system.

According to an example receiver 310 is further configured to receive from the monitoring system visual information of the patient gathered by a camera during a monitoring of the patient by at least one other sensor.

According to an example monitoring control system 300 is further configured to receive (e.g. by receiver 310) from the monitoring system questioner result information indicative of the at least one questionnaire result of a questionnaire that pertains to a sleep affecting condition of the patient and which is answered by a person. Such questionnaire results may be used, inter alia, in the analysis of the physiological monitoring information.

According to an example receiver 310 is configured to receive from the monitoring system physiological monitoring information that is responsive to information received from the multiple sensors that are of multiple types selected from a group of sensor types consisting of: electroencephalogram (EEG), electromyogram (EMG), electrocardiogram (ECG), electrooculography (EOG), and pulse sensor, microphone, etc.

According to an example receiver 310 is further configured to receive from the monitoring system location information that is responsive to information received from a positioning sensor and that is indicative of the relative location of at least one organ of the patient. Such information may be used in the analysis of the physiological monitoring information, e.g. as described above.

According to an example monitoring control system 300 includes output interface 380 configured to provide monitoring information to a medical professional, receiving from the medical professional at least one instruction (by input interface 390), and transmitting to the monitoring system an instruction to provide information on an output interface of the monitoring system in response to the instruction of the medical professional.

According to an example memory 370 (that may be internal memory, external memory, or a combination thereof) is configured to store information received from the monitoring system and/or processed/analyzed information for a monitoring duration (e.g. three days, a week, etc.) that is usually significantly longer than a period for which the monitoring system may store such information internally.

According to an example monitoring control system 300 is configured to exchange data and commands with the monitoring system, which together are used to ensure that the systems are configured properly, set-up properly, transmitting information properly, and so forth.

According to an example monitoring control system 300 (e.g. processor 320 of which) is configured to make certain that by the end of the monitoring period (and possibly during any shorter period within it), sufficient amount of usable monitoring data will be collected.

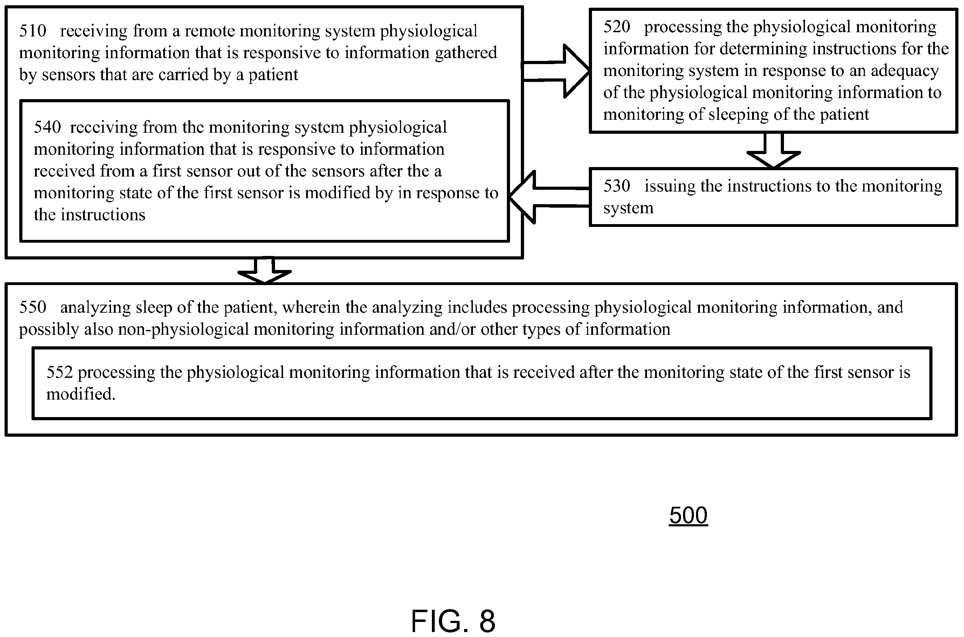

Figure 8 illustrates method 500 for controlling monitoring, according to an embodiment of the invention. Referring to the examples set forth in the previous drawings, method 500 may be carried out by monitoring control system 300. It is further noted that various embodiments of method 500 may implement all the functionalities of the various embodiments of monitoring control system 300 discussed above, even if not explicitly elaborated. The various stages of method 500 are conveniently carried out by a monitoring control system.

Method 500 includes stage 510 of receiving from a remote monitoring system physiological monitoring information that is responsive to information gathered by sensors that are carried by a patient. Referring to the examples set forth in the previous drawings, the remote monitoring system may be monitoring system 200. It is noted that the receiving may include receiving information from more than a single monitoring system, and/or information that pertains to more than a single patient. According to an example the receiving includes receiving from the monitoring system additional information (e.g. received from other sensors, status of the monitoring system itself, etc).

Method 500 further includes stage 520 of processing the physiological monitoring information for determining instructions for the monitoring system in response to an adequacy of the physiological monitoring information to monitoring of sleeping of the patient.

Stage 520 is followed by stage 530 of issuing the instructions to the monitoring system.

Stage 530 is followed by stage 540 (that may be included in stage 510) of receiving from the monitoring system physiological monitoring information that is responsive to information received from a first sensor out of the sensors after the a monitoring state of the first sensor is modified by in response to the instructions.

Method 500 continues with stage 550 of analyzing sleep of the patient, wherein the analyzing includes processing physiological monitoring information, and possibly also non-physiological monitoring information and/or other types of information.

Stage 550 includes stage 552 of processing the physiological monitoring information that is received after the monitoring state of the first sensor is modified.

According to an example method 500 further includes determining a monitoring period decision in response to an adequacy of the physiological monitoring information to monitoring of sleeping of the patient, wherein the monitoring period decision includes timing information indicative of an end of monitoring period that includes collecting of the monitoring information by the monitoring system.

According to an example method 500 further includes issuing to the monitoring system, in response to processing of the physiological monitoring information, a sensor relocating instruction for relocating at least one of the sensors that gather information for the monitoring system.

According to an example method 500 further includes receiving from the monitoring system visual information of the patient gathered by a camera during a monitoring of the patient by at least one other sensor.

According to an example method 500 further includes receiving from the monitoring system questioner result information indicative of the at least one questionnaire result of a questionnaire that pertains to a sleep affecting condition of the patient and which is answered by a person.

According to an example the receiving of the physiological monitoring information includes receiving from the monitoring system physiological monitoring information that is responsive to information received from the multiple sensors that are of multiple types selected from a group of sensor types consisting of: electroencephalogram (EEG), electromyogram (EMG), electrocardiogram (ECG), electrooculography (EOG), and pulse sensor.

According to an example method 500 further includes receiving from the monitoring system location information that is responsive to information received from a positioning sensor and that is indicative of the relative location of at least one organ of the patient.

According to an example method 500 further includes providing monitoring information to a medical professional, receiving from the medical professional at least one instruction, and transmitting to the monitoring system an instruction to provide information on an output interface of the monitoring system in response to the instruction of the medical professional.

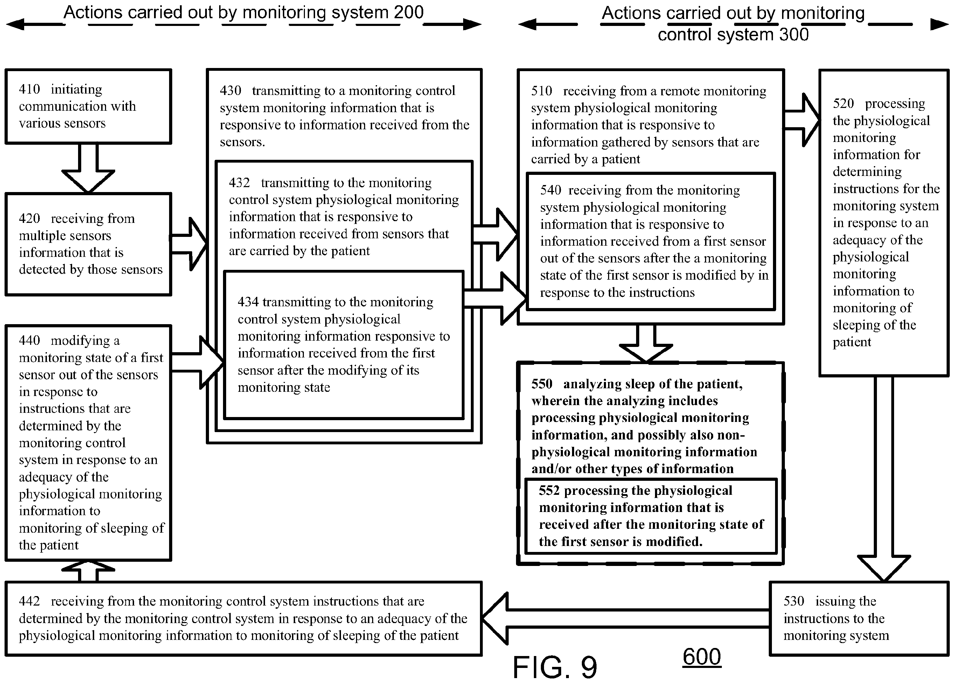

Figure 9 illustrates method 600 for monitoring, according to an embodiment of the invention. Method 600 is a combination of methods 400 and 500. It is noted that various embodiments of methods 400 and 500 may be implemented in method 600.

While certain features of the invention have been illustrated and described herein, many modifications, substitutions, changes, and equivalents will now occur to those of ordinary skill in the art. It is, therefore, to be understood that the appended claims are intended to cover all such modifications and changes as fall within the scope of the invention.

## Claims

1. A monitoring system (200), the monitoring system comprising:
a transmitter (230) configured to transmit to a monitoring control system (300) physiological monitoring information that is responsive to information received from sensors (250) that are carried by a patient; and
wherein the monitoring system is **characterized by** further comprising multiple sensor managers (210), wherein a sensor manager (210) is configured to modify a monitoring state of a first sensor out of the sensors (250) in response to instructions that are determined by the monitoring control system (300) in response to an adequacy of the physiological monitoring information to monitoring of sleeping of the patient;
the multiple sensor managers (210) configured to manage the reception of information from sensors (250), and including an internal sensor manager (210.1) and one or more external sensor managers (210.2);
wherein the transmitter (230) is further configured to transmit to the monitoring control system physiological monitoring information responsive to information received from the first sensor after the modifying of its monitoring state.

2. The monitoring system (200) according to claim 1, further comprising a monitoring timing manager (222) configured to cease, in response to a monitoring period decision that is made by the monitoring control system, a monitoring period that comprises collecting of the monitoring information transmitted to the monitoring control system; wherein the monitoring period decision is made by the monitoring control system in response to an adequacy of the physiological monitoring information to monitoring of sleeping of the patient.

3. The monitoring system (200) according to claim 1, wherein a sensor manager (210) is configured to modify the monitoring state of the first sensor by modifying a monitoring frequency of the first sensor.

4. The monitoring system (200) according to claim 1, wherein a sensor manager (210) is configured to modify the monitoring state of the first sensor by switching the first sensor ON or OFF.

5. The monitoring system (200) according to claim 1, wherein a sensor manager (210) is configured to modify the monitoring state of the first sensor by modifying a current applied to the first sensor.

6. The monitoring system (200) according to claim 1, wherein a sensor manager (210) is configured to decide about the modification of the monitoring state of the first sensor autonomously.

7. The monitoring system (200) according to claim 1, wherein the transmitter (230) is further configured to transmit to the monitoring control system location information that is responsive to information received from a positioning sensor and that is indicative of the relative location of at least one organ of the patient.

8. The monitoring system (200) according to claim 1, further comprising an interfering output interface, configured to awake the subject in response to a sleep related condition detected in response to the monitoring information.

9. A method (400) for monitoring, the method comprising carrying out by a monitoring system (200) the following stages:
transmitting (430) to a monitoring control system physiological monitoring information that is responsive to information received from sensors that are carried by a patient;
wherein the method is **characterized by** further comprising:
modifying (440), by a sensor manager (210) of the monitoring system (200), a monitoring state of a first sensor out of the sensors (250) in response to instructions that are determined by the monitoring control system (300) in response to an adequacy of the physiological monitoring information to monitoring of sleeping of the patient; and
managing by multiple sensor managers (210), including an internal sensor manager (210.1) and one or more external sensor managers (210.2), the reception of information from sensors (250); and
transmitting (434) to the monitoring control system physiological monitoring information responsive to information received from the first sensor after the modifying of its monitoring state.

10. The method (400) according to claim 9, further comprising ceasing a monitoring period that comprises collecting of the monitoring information transmitted to the monitoring control system in response to a monitoring period decision that is made by the monitoring control system in response to an adequacy of the physiological monitoring information to monitoring of sleeping of the patient

11. The method (400) according to claim 9 wherein the modifying (440) comprises modifying a monitoring frequency of the first sensor.

12. The method (400) according to claim 9 wherein the modifying comprises modifying switching the first sensor ON or OFF.

13. The method (400) according to claim 9, wherein the modifying comprises modifying a current applied to the first sensor.

14. The method (400) according to claim 9, comprising deciding autonomously by a sensor manager about the modification of the monitoring state of the first sensor.

15. The method (400) according to claim 9, further comprising transmitting to the monitoring control system location information that is responsive to information received from a positioning sensor and that is indicative of the relative location of at least one organ of the patient.

## Patentansprüche

1. Überwachungssystem (200), wobei das Überwachungssystem umfasst:
einen Sender (230), der so konfiguriert ist, dass er physiologische Überwachungsinformation als Reaktion auf Information, die von Sensoren (250) empfangen wurde, die von einem Patienten getragen werden, an ein Überwachungssteuerungssystem (300) übermittelt; und
wobei das Überwachungssystem **dadurch gekennzeichnet ist, dass** es außerdem mehrere Sensor-Manager (210) umfasst, wobei ein Sensor-Manager (210) so konfiguriert ist, dass er einen Überwachungszustand eines ersten Sensors von den Sensoren (250) als Antwort auf Befehle, die durch das Überwachungssteuerungssystem (300) als Antwort auf eine Adäquanz der physiologischen Überwachungsinformation zur Überwachung des Schlafes des Patienten festgelegt werden, modifiziert;
die mehreren Sensor-Manager (210), die so konfiguriert sind, dass sie den Empfang von Information von den Sensoren (250) verwalten, und einen internen Sensor-Manager (210.1) und einen oder mehrere externe Sensor-Manager (210.2) umfassen;
wobei der Sender (230) außerdem so konfiguriert ist, dass er physiologische Überwachungsinformation als Reaktion auf Information, die von dem ersten Sensor nach der Modifizierung seines Überwachungszustands empfangen wurde, an das Überwachungssteuerungssystem übermittelt.

2. Überwachungssystem (200) gemäß Anspruch 1, welches außerdem einen Überwachungs-Timing-Manager (222) umfasst, der so konfiguriert ist, dass er, als Antwort auf eine durch das Überwachungssteuerungssystem getroffene Überwachungszeitraum-Entscheidung, einen Überwachungszeitraum, welcher das Sammeln der dem Überwachungssteuerungssystem übermittelten Überwachungsinformation umfasst, beendet; wobei die Überwachungszeitraum-Entscheidung durch das Überwachungssteuerungssystem als Antwort auf eine Adäquanz der physiologischen Überwachungsinformation zur Überwachung des Schlafes des Patienten getroffen wird.

3. Überwachungssystem (200) gemäß Anspruch 1, wobei ein Sensor-Manager (210) so konfiguriert ist, dass er den Überwachungszustand des ersten Sensors durch Modifizieren einer Überwachungsfrequenz des ersten Sensors modifiziert.

4. Überwachungssystem (200) gemäß Anspruch 1, wobei ein Sensor-Manager (210) so konfiguriert ist, dass er den Überwachungszustand des ersten Sensors durch An- oder Ausschalten des ersten Sensors modifiziert.

5. Überwachungssystem (200) gemäß Anspruch 1, wobei ein Sensor-Manager (210) so konfiguriert ist, dass er den Überwachungszustand des ersten Sensors durch Modifizieren eines an den ersten Sensor angelegten Stroms modifiziert.

6. Überwachungssystem (200) gemäß Anspruch 1, wobei ein Sensor-Manager (210) so konfiguriert ist, dass er über die Modifikation des Überwachungszustands des ersten Sensors autonom entscheidet.

7. Überwachungssystem (200) gemäß Anspruch 1, wobei der Sensor (230) außerdem so konfiguriert ist, dass er Lageinformation als Reaktion auf Information, die von einem Positionierungssensor empfangen wurde und die die relative Lage wenigstens eines Organs des Patienten anzeigt, an das Überwachungssteuerungssystem übermittelt.

8. Überwachungssystem (200) gemäß Anspruch 1, welches außerdem eine interferierende Ausgabeschnittstelle umfasst, die so konfiguriert ist, dass die Person als Antwort auf einen mit dem Schlaf zusammenhängenden Zustand, der als Antwort auf die Überwachungsinformation detektiert wurde, geweckt wird.

9. Überwachungsverfahren (400) wobei das Verfahren das Durchführen der folgenden Schritte durch ein Überwachungssystem (200) umfasst:
Übermitteln (430) von physiologischer Überwachungsinformation als Reaktion auf Information, die von Sensoren empfangen wurde, die von einem Patienten getragen werden, an ein Überwachungssteuerungssystem;
wobei das Verfahren **dadurch gekennzeichnet ist, dass** es außerdem umfasst:
Modifizieren (440), durch einen Sensor-Manager (210) des Überwachungssystems (200), eines Überwachungszustands eines ersten Sensors von den Sensoren (250) als Antwort auf Befehle, die durch das Überwachungssteuerungssystem (300) als Antwort auf eine Adäquanz der physiologischen Überwachungsinformation zur Überwachung des Schlafes des Patienten festgelegt werden; und
Verwalten, durch mehrere Sensor-Manager (210), die einen internen Sensor-Manager (210.1) und einen oder mehrere externe Sensor-Manager (210.2) umfassen, des Empfangs von Information von Sensoren (250); und
Übermitteln (434) von physiologischer Überwachungsinformation als Reaktion auf Information, die von dem ersten Sensor nach der Modifizierung seines Überwachungszustands empfangen wurde, an das Überwachungssteuerungssystem.

10. Verfahren (400) gemäß Anspruch 9, welches außerdem das Beenden eines Überwachungszeitraums, welcher das Sammeln der dem Überwachungssteuerungssystem übermittelten Überwachungsinformation umfasst, als Antwort auf eine Überwachungszeitraum-Entscheidung, die durch das Überwachungssteuerungssystem als Antwort auf eine Adäquanz der physiologischen Überwachungsinformation zur Überwachung des Schlafes des Patienten getroffen wird, umfasst.

11. Verfahren (400) gemäß Anspruch 9, wobei das Modifizieren (440) das Modifizieren einer Überwachungsfrequenz des ersten Sensors umfasst.

12. Verfahren (400) gemäß Anspruch 9, wobei das Modifizieren das Modifizieren des Ein- oder Ausschaltens des ersten Sensors umfasst.

13. Verfahren (400) gemäß Anspruch 9, wobei das Modifizieren das Modifizieren eines an den ersten Sensor angelegten Stroms umfasst.

14. Verfahren (400) gemäß Anspruch 9, welches das autonome Entscheiden über die Modifikation des Überwachungszustands des ersten Sensors durch einen Sensor-Manager umfasst.

15. Verfahren (400) gemäß Anspruch 9, welches außerdem die Übermittlung von Lageinformation als Reaktion auf Information, die von einem Positionierungssensor empfangen wurde und die die relative Lage wenigstens eines Organs des Patienten anzeigt, an das Überwachungssteuerungssystem umfasst.

## Revendications

1. Système de surveillance (200), le système de surveillance comprenant :
un émetteur (230) configuré pour émettre, vers un système de commande de surveillance (300), des informations de surveillance physiologiques en réaction à des informations reçues de capteurs (250) qui sont portés par un patient ; et
dans lequel le système de surveillance est **caractérisé en ce qu'**il comprend en outre plusieurs dispositifs de gestion de capteur (210), dans lequel un dispositif de gestion de capteur (210) est configuré pour modifier un état de surveillance d'un premier capteur parmi les capteurs (250) en réaction à des instructions qui sont déterminées par le système de commande de surveillance (300) en réaction à une adéquation des informations de surveillance physiologiques avec une surveillance du sommeil du patient ;
les plusieurs dispositifs de gestion de capteur (210) étant configurés pour gérer la réception d'informations en provenance de capteurs (250), et comprenant un dispositif de gestion de capteurs interne (210.1) et un ou plusieurs dispositif(s) de gestion de capteurs externe(s) (210.2) ;
dans lequel l'émetteur (230) est en outre configuré pour émettre, vers le système de commande de surveillance, des informations de surveillance physiologiques en réaction à des informations reçues du premier capteur après l'étape de modification de son état de surveillance.

2. Système de surveillance (200) selon la revendication 1, comprenant en outre un dispositif de gestion de temporisation de surveillance (222) configuré pour interrompre, en réaction à une décision de période de surveillance qui est prise par le système de commande de surveillance, une période de surveillance qui comprend une collecte des informations de surveillance émises vers le système de commande de surveillance ; dans lequel la décision de période de surveillance est prise par le système de commande de surveillance en réaction à une adéquation des informations de surveillance physiologiques avec une surveillance du sommeil du patient.

3. Système de surveillance (200) selon la revendication 1, dans lequel un dispositif de gestion de capteur (210) est configuré pour modifier l'état de surveillance du premier capteur grâce à une étape consistant à modifier une fréquence de surveillance du premier capteur.

4. Système de surveillance (200) selon la revendication 1, dans lequel un dispositif de gestion de capteur (210) est configuré pour modifier l'état de surveillance du premier capteur grâce à une étape consistant à commuter le premier capteur sur MARCHE ou ARRÊT.

5. Système de surveillance (200) selon la revendication 1, dans lequel un dispositif de gestion de capteur (210) est configuré pour modifier l'état de surveillance du premier capteur grâce à une étape consistant à modifier un courant appliqué au premier capteur.

6. Système de surveillance (200) selon la revendication 1, dans lequel un dispositif de gestion de capteur (210) est configuré pour décider de la modification de l'état de surveillance du premier capteur de manière autonome.

7. Système de surveillance (200) selon la revendication 1, dans lequel l'émetteur (230) est en outre configuré pour émettre, vers le système de commande de surveillance, des informations d'emplacement en réaction à des informations reçues d'un capteur de positionnement et indicatives de l'emplacement relatif d'au moins un organe du patient.

8. Système de surveillance (200) selon la revendication 1, comprenant en outre une interface de sortie interférente configurée pour réveiller le sujet en réaction à un état lié au sommeil et détecté en réaction aux informations de surveillance.

9. Procédé (400) de surveillance, le procédé comprenant une mise en oeuvre, par un système de surveillance (200), des étapes ci-dessous :
émission (430), vers un système de commande de surveillance, d'informations de surveillance physiologiques en réaction à des informations reçues de capteurs qui sont portés par un patient ;
dans lequel le procédé est **caractérisé en ce qu'**il comprend en outre :
modification (440), par un dispositif de gestion de capteur (210) du système de surveillance (200), d'un état de surveillance d'un premier capteur parmi les capteurs (250) en réaction à des instructions qui sont déterminées par le système de commande de surveillance (300) en réaction à une adéquation des informations de surveillance physiologiques avec une surveillance du sommeil du patient ; et
gestion par plusieurs dispositifs de gestion de capteur (210), comprenant un dispositif de gestion de capteurs interne (210.1) et un ou plusieurs dispositif(s) de gestion de capteurs externe(s) (210.2), de la réception d'informations par des capteurs (250) ; et
émission (434), vers le système de commande de surveillance, d'informations de surveillance physiologiques en réaction à des informations reçues du premier capteur après la modification de son état de surveillance.

10. Procédé (400) selon la revendication 9, comprenant en outre une interruption d'une période de surveillance qui comprend une collecte des informations de surveillance émises vers le système de commande de surveillance en réaction à une décision de période de surveillance qui est prise par le système de commande de surveillance en réaction à une adéquation des informations de surveillance physiologiques avec la surveillance du sommeil du patient.

11. Procédé (400) selon la revendication 9, dans lequel la modification (440) comprend une modification d'une fréquence de surveillance du premier capteur.

12. Procédé (400) selon la revendication 9, dans lequel la modification comprend une modification de la commutation du premier capteur sur MARCHE ou ARRÊT.

13. Procédé (400) selon la revendication 9, dans lequel la modification comprend une modification d'un courant appliqué au premier capteur.

14. Procédé (400) selon la revendication 9, comprenant une décision de manière autonome par un dispositif de gestion de capteur quant à la modification de l'état de surveillance du premier capteur.

15. Procédé (400) selon la revendication 9, comprenant en outre une émission, vers le système de commande de surveillance, d'informations d'emplacement en réaction à des informations reçues d'un capteur de positionnement et indicatives de l'emplacement relatif d'au moins un organe du patient.
